# EUROPEAN PATENT SPECIFICATION

(11) **EP 0 820 276 B1**
(45) Date of publication and mention of the grant of the patent: **02.01.2003**
(21) Application number: 96910285.4
(22) Date of filing: 12.04.1996
(51) Int. Cl.: A61K 9/14, B01D 9/02, A61K 9/72

(54) **PROCESS FOR THE PREPARATION OF RESPIRABLE PARTICLES**
VERFAHREN ZUR HERSTELLUNG EINATEMBARER PARTIKEL
PROCEDE DE PREPARATION DE PARTICULES RESPIRABLES

(30) Priority: 13.04.1995 SE 9501384
(43) Date of publication of application: 28.01.1998
(73) Proprietor: AstraZeneca AB, 151 85 Södertälje (SE)
(72) Inventor: JAKUPOVIC, Edib, S-155 31 Nykvarn (SE); TROFAST, Jan, S-226 47 Lund (SE)
(86) International application number: PCT/SE96/00479
(87) International publication number: WO 96/032095

(56) References cited:
- EP-A- 0 169 618
- EP-A- 0 677 332
- WO-A-90/03782
- US-A- 5 314 506

## Description

### Field of the invention

This invention relates to a process for preparing a pharmaceutical powder for inhalation, which powder comprises crystalline particles of an inhalation compound, in particular particles of mass median diameter 10µm or less.

### Background of the invention

Inhalation of dry powders has been recognised as a valuable method for the administration of pharmacological agents and in particular those which are useful in the treatment of diseases of the respiratory tract. However, the utility of the method has been limited by the difficulty in making appropriate doses available to the lower respiratory tract of the patient. In general only a relatively small proportion of any nominal dose will reach the lower respiratory tract of the inhaling patient; the remainder may remain in the inhaler device or be deposited in the mouth and throat of the patient.

One major factor determining the proportion of inhalable drug which will reach the lower respiratory tract of a patient is the particle size distribution of the particles emerging from the inhaler device. This particle size distribution is in turn dependent both on the construction and function of the inhaler, and the powder formulation. The present application is concerned with the nature of the powder formulation. This should have a high integrity of the crystal structure, or crystal habit (as may be measured using X-ray crystallography techniques, for example), high purity and stability, and a particle size within the respirable particle range.

In order to achieve a desired crystal structure and particle size, powder formulations of respirable particles are in general obtained by processes including crystallisation from solution followed by micronisation. However, optimum crystal structure formation and optimum purity may not be obtained in this process, and micronisation has associated problems. Prolonged comminution results in a high energy powder and crystal lattice defects which may be manifested for example in lower stability, and/or hygroscopicity. As micronisation results in some amorphous character on the surface of the obtained particles, a "conditioning" step is necessary in order to obtain a particle considered to be completely crystalline. International patent applications PCT/SE91/00186 (WO 92/18110) and PCT/SE94/00780 (WO95/05805) describe methods of conditioning substances in order to obtain crystalline products. It is an object of the present invention to provide a process for the production of crystalline respirable particles which avoids the necessity for post-crystallisation micronisation.

European Patent no. 437451 discloses a process for producing finely divided solid crystalline powders, comprising dissolving the solid to be finely divided in a liquid carrier solvent to form an injection solution and adding the injection solution to a volume of anti-solvent, which is a supercritical fluid, liquefied compressed gas or dense vapour, sufficient to precipitate or crystallise the solid.

European patent application, publication number 0 542 314 A1, discloses a method of forming microparticles of a material, involving bringing a supercritical anti-solvent gas into contact with a solution of said material in a solvent at a controlled rate operable to expand the solution and precipitate the material. Needles and globules are formed.

Neither of the above are directed to powders for inhalation per se, and as they use supercritical media the use of compressed gases and heavy, expensive apparatus is necessitated.

International patent application, publication number WO90/03782 discloses a process for producing a finely divided solid, involving dissolving the solid to be finely divided in a solvent and adding the resulting solution to an anti-solvent to precipitate or crystallise the solid. The anti-solvents include supercritical fluids, compressed liquified gases or dense vapours.

European patent application, publication number EP0677332, describes dissolving a substance in a fluid to form a solution and mixing the solution with a second fluid to form fine particles. The second fluid includes supercritical fluids.

US Patent number 5 314 506, again not related to powders for inhalation, describes impinging a jet stream of an organic pharmaceutical compound and a jet stream of an antisolvent, to precipitate small crystals of the organic pharmaceutical compound. Pressurised blow-cans and elevated temperatures are employed, and the crystals obtained range from flakes of up to 25 microns, to needles, and cubes of less than 3 microns.

EP 0169618 discloses a method for the preparation of water-insoluble organic compounds such as may be used in suspensions for intravenous injection, as non-crystalline particles. The method involves preparing a solution of the compound in a water-miscible organic solvent and infusing an aqueous precipitating liquid into the organic solution, in most cases in the presence of surfactant.

The process of the present invention aims to provide a powder for inhalation comprising crystalline particles of an inhalation compound of mass median diameter less than 10 µm, irrespective of the substance concerned. Thus the process does not require the use of supercritical media nor the processes of micronising and conditioning.
According to the present invention there is provided a process for producing a pharmaceutical powder for inhalation comprising crystalline particles of an inhalation compound, comprising dissolving an inhalation compound to be provided in crystalline particle form in a solvent; and introducing the solution containing the inhalation compound, in droplet form or as a jet stream, into an anti-solvent which is miscible with the solvent and which is under agitation, under non-supercritical conditions.

Preferably the particles of the inhalation compound of the present invention have a mass median diameter of at least 0.1 µm, more preferably at least 1 µm. Where the powder is intended particularly for oral inhalation, preferably the particles have a mass median diameter of 10µm or less, preferably 7µm or less. Most preferably the particles have a mass median diameter of 1-6µm. By "mass median diameter" is meant that half of the mass of the inhalation compound is made up of particles having a diameter less than the mass median diameter and half of the mass of the inhalation compound is made up of particles having a diameter greater than the mass median diameter. Preferably as much as possible of the powder consists of particles of diameter 10µm or less; for example at least 75% or at least 90% of the powder consists of particles of diameter 10µm or less.

The pharmaceutical powder of the present invention may be administered orally or nasally. Where nasal administration is intended the particles of the inhalation compound may have a mass median diameter outside the above preferred ranges.

The pharmaceutical powder may comprise a water-soluble inhalation compound, or a water-insoluble inhalation compound.

The solution containing the inhalation compound is introduced into the anti-solvent in droplet form or as a jet-stream, for example through a porous filter or one or more nozzles.

Through the present invention it is possible to control the size of particles obtained by controlling any or all of parameters such as the concentration of the compound in the solvent, the rate of addition of the solution into the anti-solvent and the intensity of the agitation such that particles within a specific desired particle size range may be obtained. A powder formulation having good physicochemical stability and needing no mechanical micronisation or conditioning is obtained.

Medically useful compounds which may be provided in respirable particle form according to the present invention include β2-adrenoreceptor agonists, for example salbutamol, terbutaline, rimiterol, fenoterol, reproterol, adrenaline, pirbuterol, isoprenaline, orciprenaline, bitolterol, salmeterol, formoterol, clenbuterol procaterol, broxaterol, picumeterol, TA-2005 ([8-hydroxy-5-(1-hydroxy-2-((2-(4-methoxyphenyl)-1-methylethyl)amino)ethyl)]-2(1H)-quinolone), mabuterol; anticholinergic bronchodilators, for example ipratropium bromide; glucocorticosteroids, for example betamethasone, fluticasone, budesonide, tipredane, dexamethasone, beclomethasone, fluocinolone, triamcinolone acetonide, mometasone and rofleponide; peptides and proteins, for example insulin, immunomodulators, anti-allergic drugs for example sodium cromoglycate and nedocromil sodium; expectorants; mucolytics; antihistamines; cyclooxygenase inhibitors; leukotriene synthesis inhibitors; leukotriene antagonists, PLA2 inhibitors, PKC-inhibitors, PAF antagonists and prophylactics of asthma; or pharmacologically acceptable esters and salts and/or solvates thereof. For example salbutamol and terbutaline may be used as the sulphate; fenoterol as the hydrobromide; salmeterol as the xinafoate; formoterol as the fumarate dihydrate; clenbuterol as the hydrochloride; fluticasone as the propionate; and broxaterol as the monohydrochloride.

Preferably the medically useful compound is selected from β2-adrenoreceptor agonists and glucocorticosteroids.

Most preferably the medically useful compound is selected from salbutamol, preferably as the sulphate, salmeterol, preferably as the xinafoate, formoterol, preferably as the fumarate dihydrate, budesonide, terbutaline, preferably as the sulphate, fluticasone, preferably as the propionate, rofleponide, preferably as the palmitate, and other pharmaceutically acceptable esters and salts and/or solvates thereof.

Pharmaceutically acceptable additives e.g., carriers, diluents, and penetration enhancers may also be prepared according to the present invention. For example the process of the present invention may be used to prepare carbohydrates such as lactose, dextrose, melezitose, maltose, mannitol, trehalose and raffinose, as well as salts of fatty acids, bile salts, phospholipids and alkyl glycosides, which may be useful as penetration enhancers. The pharmaceutically acceptable additive may be prepared separately from the medically useful compound, and the powders may then be mixed together, or a powder containing the medically useful compound and additive may be prepared in certain cases, i.e. when the compound and additive have similar solubility's, by dissolving all of the desired substances together in the solvent according to the present invention.

In the process of the present invention, the choice of solvent depends upon the solubility of the compound to be dissolved. Preferably, a substantially saturated or supersaturated solution is obtained. The anti-solvent should be miscible with the solvent in order that a single-phase solvent mixture is formed and should be such that the dissolved compound is precipitated immediately upon contact therewith.

The choice of particular solvent and anti-solvent can be made readily by a person skilled in the art considering the solubility characteristics of the compound to be precipitated. In general, water-soluble substances may be dissolved in water or another solvent more polar than the anti-solvent, or a mixture of such solvents, and precipitated with a less polar solvent (the anti-solvent); and substantially water-insoluble substances may be dissolved in a less polar solvent and precipitated with water or another "more polar" solvent (the anti-solvent). For example, a water-soluble substance which is dissolved for example in water may be precipitated with an anti-solvent such as ethyl acetate, acetone, methylethyl ketone (2-butanone), isopropanol, or mixtures of for example 10-20% methanol, isopropanol or ethanol with 80-90% (w/w) methylethyl ketone or isopropanol, while a less water-soluble substance may be dissolved for example in an organic solvent such as methanol, isopropanol or another alcohol, dimethyl sulphoxide, dimethyl formamide, N'N'-dimethyl acetamide or phenol and precipitated with for example water.

To maximise the degree of precipitation it is desirable that the solution is added to the anti-solvent at temperatures as low as possible, but low temperatures are not essential for the process of the present invention. Preferably, the solution is added to the anti-solvent at temperatures of below 25 °C, for example at around 0°C or from 0 to 5 °C.

According to the present invention the solution is preferably added to the anti-solvent through a porous filter having pores of 10 - 160 microns, such as Pyrex Glass Filters of porosity grades 1-4.

The rate of addition may be controlled, for example by using a pump, such as a peristaltic pump when working on a laboratory scale.

In order that the droplet or fine jet, as well as the precipitated compound, is efficiently removed from the porous filter or nozzle, it is necessary for the anti-solvent to be under agitation. The agitation may be achieved in various ways such as by means of mechanical stirring with propellers, turbines, paddles, anchor impellers or Ystral equipment, or by using ultrasound waves on or beside the filter or nozzles.

The precipitated compound may be dried in conventional manner, for example it may be spray-dried, and may be agglomerated and/or spheronised if desired. No conditioning is necessary as the particles obtained are considered to be completely crystalline.

The size of the particles obtained according to the present process may be controlled by adjusting the process parameters, as will be evident to a person skilled in the art. For example, decreasing the concentration of compound in the solvent will lead to smaller particles, and adjusting the rate of addition and/or agitation will alter the particle size by altering the size of the droplet from which the compound is precipitated. Any one, several, or all of the process parameters may be adjusted in order to obtain a particular particle size range. The optimal process parameters in each case may be determined by a person skilled in the art using routine experimentation.

Various methods may be employed in order to monitor the crystallinity of the respirable particles of the present invention. Such methods, which are all well known to the skilled person in the art, include isothermal micro calorimetry, BET gas adsorption, X-ray powder diffraction and differential scanning calorimetry (DSC). For example during a recrystallisation a large amount of heat is evolved and by monitoring the calometrical signal the sample may be checked for any amorphous content.

The following Examples are intended to illustrate the present invention. The particle sizes given were measured using a Malvern Master Sizer instrument.

### Example 1

A solution of budesonide (15 g) in methanol (300 ml) was added to water (450 ml) at a temperature of 0°C, through a glass filter, Pyrex porosity grade 3 (pore index 16-40 microns), and with stirring with ultraturrax equipment.

A slurry was obtained, containing particles with a mass median diameter (MMD) of 2.2 microns. 90% of the particles had a diameter of below 5.3µm. The slurry was spray dried using a commercially available spray-dryer (Büchi 190), to give a budesonide powder consisting of particles of MMD 2.9 microns having no amorphous character, i.e., crystalline particles, determined using powder X-ray measurements. 90% of the particles had a diameter of below 5.7µm.

The particles obtained could be agglomerated for use in dry powder inhalers.

### Example 2

A solution of budesonide (2.35 g) in methanol (60 ml) was added to water/ice (200 ml) at a rate of 1 ml/min, through a glass filter with a porosity of 40-90 microns, and with stirring with ultraturrax equipment (4.5). The obtained slurry contained budesonide crystalline particles of MMD 2.79 microns. 90% of the particles had a diameter of below 6.0µm.

### Example 3

A solution of budesonide (1.5 g) in methanol (80 ml) was added to water/ice (300 ml) at a rate of 1 ml/min, through a Pyrex glass filter of porosity grade 2 (40-100 microns), and with stirring with ultraturrax equipment (4.5). The obtained slurry contained budesonide crystalline particles of MMD 2.60 microns. 90% of the particles had a diameter of below 6.0µm.

### Example 4

The procedure of Example 3 was repeated using a Pyrex glass filter of porosity grade 4(10-16 microns). The obtained slurry contained budesonide crystalline particles of MMD 2.49 microns. 90% of the particles had a diameter of below 6.0µm.

### Example 5

A solution of budesonide (3 g) in methanol (90 ml) was added to water/ice (300 ml) at a rate of 1 ml/min, through a Pyrex glass filter of porosity grade 1 (100-160 microns), and with stirring with ultraturrax equipment (4.5). The obtained slurry contained budesonide crystalline particles of MMD 4.76 microns. 90% of the particles had a diameter of below 9.6µm.

### Example 6

A solution of budesonide (1.2 g) in methanol (50 ml) was added via a peristaltic pump and through a glass filter, Pyrex no. 3 (16-40 microns), to water (200 ml) at 0°C and with stirring with Ystral agitation equipment. The rate of addition was 3 ml/min and the rate of stirring was 1500 r/min. The slurry obtained contained budesonide crystalline particles of MMD 4.2 microns. 90% of the particles had a diameter of below 8.6µm.

### Example 7

A solution of lactose (2.0 g) in water was added via a peristaltic pump and through a glass filter, Pyrex no. 3, to a 20% solution of ethanol in methylethyl ketone, with stirring with Ystral agitation equipment. The rate of addition was 3 ml/min and the rate of stirring was 1500 r/min. The slurry obtained contained lactose particles of MMD 5.2 microns. 75% of the particles had a diameter of below 10.0µm

### Example 8

A solution of salbutamol sulphate (1 g) in water (7 ml) was added via a peristaltic pump and through a glass filter, Pyrex no. 2, to a 20% solution of ethanol in methylethyl ketone at room temperature, with stirring with Ystral agitation equipment. The rate of addition was 3 ml/min and the rate of stirring was 1500 r/min. The slurry obtained contained salbutamol sulphate crystalline particles of MMD 5.2 microns. 75% of the particles had a diameter of below 10.0µm.

## Claims

1. A process for producing a pharmaceutical powder for inhalation comprising crystalline particles of an inhalation compound, comprising dissolving an inhalation compound in a solvent; and introducing the solution containing the inhalation compound in droplet form or as a jet stream, into an anti-solvent which is miscible with the solvent and which is under agitation, under non-supercritical conditions.

2. A process as claimed in claim 1 wherein the particles of the inhalation compound have a mass median diameter of 10µm or less.

3. A process as claimed in claim 1 wherein the particles of the inhalation compound have a mass median diameter of 7µm or less.

4. A process as claimed in claim 1 wherein the particles of the inhalation compound have a mass median diameter of at least 0.1µm.

5. A process as claimed in claim 1 wherein the particles of the inhalation compound have a mass median diameter of at least 1µm.

6. A process as claimed in claim 1 wherein the particles of the inhalation compound have a mass median diameter of 1-6µm.

7. A process as claimed in claim 1 wherein at least 75% of the powder consists of particles having a diameter of 10µm or less.

8. A process as claimed in claim 1 wherein at least 90% of the powder consists of particles having a diameter of 10µm or less.

9. A process as claimed in any preceding claim wherein the solution containing the inhalation compound is introduced into the anti-solvent through a porous filter.

10. A process as claimed in any preceding claim wherein the solution containing the inhalation compound is introduced into the anti-solvent through one or more nozzles.

11. A process as claimed in any preceding claim wherein the solution containing the inhalation compound is substantially saturated or supersaturated.

12. A process as claimed in any preceding claim wherein the inhalation compound is selected from β2-adrenoreceptor agonists, anticholinergic bronchodilators, glucocorticosteroids, immunomodulators, anti-allergic drugs, expectorants, mucolytics, antihistamines, cyclooxygenase inhibitors, leukotriene synthesis inhibitors, leukotriene antagonists, PLA2 inhibitors, PKC-inhibitors, PAF antagonists and prophylactics of asthma, and pharmacologically acceptable esters and salts and/or hydrates thereof; and pharmaceutically acceptable additives.

13. A process as claimed in claim 12 wherein the inhalation compound is selected from β2-adrenoreceptor agonists and glucocorticosteroids.

14. A process as claimed in claim 13 wherein the inhalation compound is selected from salbutamol, salmeterol, formoterol, budesonide, terbutaline, fluticasone, rofleponide, and pharmaceutically acceptable esters and salts and/or solvates thereof.

15. A process as claimed in claim 14 wherein the inhalation compound is salbutamol sulphate.

16. A process as claimed in claim 14 wherein the inhalation compound is formoterol fumarate dihydrate.

17. A process as claimed in claim 14 wherein the inhalation compound is budesonide.

18. A process as claimed in claim 14 wherein the inhalation compound is terbutaline sulphate.

19. A process as claimed in claim 14 wherein the inhalation compound is rofleponide palmitate.

20. A process as claimed in claim 14 wherein the inhalation compound is salmeterol xinafoate.

21. A process as claimed in any of claims 1-11, wherein the inhalation compound is a pharmaceutically acceptable additive.

22. A process as claimed in any of claims 1-11 wherein the inhalation compound is water-soluble, the solvent is water or another polar solvent or mixture of polar solvents, and the anti-solvent is a less polar solvent.

23. A process as claimed in claim 22 wherein the anti-solvent is selected from ethyl acetate, acetone, methylethyl ketone, isopropanol or mixtures of 10-20% methanol, isopropanol or ethanol with 80-90% methylethyl ketone or isopropanol.

24. A process as claimed in any of claims 1-11 wherein the inhalation compound is substantially water-insoluble, the solvent is less polar than the anti-solvent, and the anti-solvent is water or another polar solvent or mixture of polar solvents.

25. A process as claimed in claim 24 wherein the solvent is selected from methanol, isopropanol, dimethylsulphoxide, dimethylformamide, N'NT-dimethyl acetamide and phenol.

26. A process as claimed in any preceding claim wherein the solution containing the inhalation compound is added to the anti-solvent at a temperature of below 25°C.

27. A process as claimed in claim 26 wherein the solution containing the inhalation compound is added to the anti-solvent at a temperature of from 0°to 5°C.

28. A process as claimed in any preceding claim wherein agitation of the anti-solvent is achieved by means of mechanical stirring, propellers, turbines paddles, anchor impellers of Ystral equipment, or by using ultrasound waves on or beside the filter or nozzles.

## Patentansprüche

1. Verfahren zur Herstellung eines pharmazeutischen Pulvers zur Inhalation, welches kristalline Teilchen einer Verbindung zur Inhalation enthält, bei dem man eine Verbindung zur Inhalation in einem Lösungsmittel löst und die die Verbindung zur Inhalation enthaltende Lösung in Tröpfchenform oder als Strahl in ein Antilösungsmittel einbringt, das mit dem Lösungsmittel mischbar ist und unter nichtüberkritischen Bedingungen gerührt wird.

2. Verfahren nach Anspruch 1, bei dem die Teilchen der Verbindung zur Inhalation einen massenmittleren Durchmesser von 10 µm oder weniger aufweisen.

3. Verfahren nach Anspruch 1, bei dem die Teilchen der Verbindung zur Inhalation einen massenmittleren Durchmesser von 7 µm oder weniger aufweisen.

4. Verfahren nach Anspruch 1, bei dem die Teilchen der Verbindung zur Inhalation einen massenmittleren Durchmesser von wenigstens 0,1 µm aufweisen.

5. Verfahren nach Anspruch 1, bei dem die Teilchen der Verbindung zur Inhalation einen massenmittleren Durchmesser von wenigstens 1 µm aufweisen.

6. Verfahren nach Anspruch 1, bei dem die Teilchen der Verbindung zur Inhalation einen massenmittleren Durchmesser von 1-6 µm aufweisen.

7. Verfahren nach Anspruch 1, bei dem wenigstens 75% des Pulvers aus Teilchen mit einem Durchmesser von 10 µm oder weniger bestehen.

8. Verfahren nach Anspruch 1, bei dem wenigstens 90% des Pulvers aus Teilchen mit einem Durchmesser von 10 µm oder weniger bestehen.

9. Verfahren nach einem der vorhergehenden Ansprüche, bei dem man die die Verbindung zur Inhalation enthaltende Lösung durch einen porösen Filter in das Antilösungsmittel einbringt.

10. Verfahren nach einem der vorhergehenden Ansprüche, bei dem man die die Verbindung zur Inhalation enthaltende Lösung durch eine oder mehrere Düsen in das Antilösungsmittel einbringt.

11. Verfahren nach einem der vorhergehenden Ansprüche, bei dem die die Verbindung zur Inhalation enthaltende Lösung im wesentlichen gesättigt oder übersättigt ist.

12. Verfahren nach einem der vorhergehenden Ansprüche, bei dem die Verbindung zur Inhalation ausgewählt ist aus β2-Adrenorezeptoragonisten, anticholinergen Bronchodilatoren, Glucocorticosteroiden, Immunmodulatoren, antiallergischen Arzneimitteln, Expektoranzien, Mukolytika, Antihistaminen, Cyclooxygenaseinhibitoren, Leukotriensyntheseinhibitoren, Leukotrienantagonisten, PLA2-Inhibitoren, PKC-Inhibitoren, PAF-Antagonisten und Asthmaprophylaktika sowie deren pharmakologisch unbedenklichen Estern und Salzen und/oder Hydraten, und pharmazeutisch unbedenklichen Zusatzstoffen.

13. Verfahren nach Anspruch 12, bei dem die Verbindung zur Inhalation ausgewählt ist aus •2-Adrenorezeptoragonisten und Glucocorticosteroiden.

14. Verfahren nach Anspruch 13, bei dem die Verbindung zur Inhalation ausgewählt ist aus Salbutamol, Salmeterol, Formoterol, Budenosid, Terbutalin, Fluticason, Rofleponid und deren pharmazeutisch unbedenklichen Estern und Salzen und/oder Solvaten.

15. Verfahren nach Anspruch 14, bei dem es sich bei der Verbindung zur Inhalation um Salbutamolsulfat handelt.

16. Verfahren nach Anspruch 14, bei dem es sich bei der Verbindung zur Inhalation um Formoterolfumarat-Dihydrat handelt.

17. Verfahren nach Anspruch 14, bei dem es sich bei der Verbindung zur Inhalation um Budesonid handelt.

18. Verfahren nach Anspruch 14, bei dem es sich bei der Verbindung zur Inhalation um Terbutalinsulfat handelt.

19. Verfahren nach Anspruch 14, bei dem es sich bei der Verbindung zur Inhalation um Rofleponidpalmitat handelt.

20. Verfahren nach Anspruch 14, bei dem es sich bei der Verbindung zur Inhalation um Salmeterolxinafoat handelt.

21. Verfahren nach einem der Ansprüche 1-11, bei dem es sich bei der Verbindung zur Inhalation um einen pharmakologisch unbedenklichen Zusatzstoff handelt.

22. Verfahren nach einem der Ansprüche 1-11, bei dem die Verbindung zur Inhalation wasserlöslich ist und es sich bei dem Lösungsmittel um Wasser oder ein anderes polares Lösungsmittel oder eine Mischung polarer Lösungsmittel und bei dem Antilösungsmittel um ein weniger polares Lösungsmittel handelt.

23. Verfahren nach Anspruch 22, bei dem das Antilösungsmittel aus Essigsäureethylester, Aceton, Methylethylketon, Isopropanol oder Mischungen von 10-20% Methanol, Isopropanol oder Ethanol mit 80-90% Methylethylketon oder Isopropanol ausgewählt ist.

24. Verfahren nach einem der Ansprüche 1-11, bei dem die Verbindung zur Inhalation im wesentlichen wasserunlöslich ist, das Lösungsmittel weniger polar als das Antilösungsmittel ist und es sich bei dem Antilösungsmittel um Wasser oder ein anderes polares Lösungsmittel oder eine Mischung polarer Lösungsmittel handelt.

25. Verfahren nach Anspruch 24, bei dem das Lösungsmittel ausgewählt ist aus Methanol, Isopropanol, Dimethylsulfoxid, Dimethylformamid, N,N-Dimethylacetamid und Phenol.

26. Verfahren nach einem der vorhergehenden Ansprüche, bei dem man die die Verbindung zur Inhalation enthaltende Lösung dem Antilösungsmittel bei einer Temperatur von unter 25°C zusetzt.

27. Verfahren nach Anspruch 26, bei dem man die die Verbindung zur Inhalation enthaltende Lösung dem Antilösungsmittel bei einer Temperatur von 0°-5°C zusetzt.

28. Verfahren nach einem der vorhergehenden Ansprüche, bei dem man das Rühren des Antilösungsmittels durch mechanisches Rühren, durch Propeller, durch Turbinenschaufeln, durch Ankerrührer von Ystral-Ausrüstungen oder durch die Anwendung von Schallwellen auf oder neben dem Filter oder den Düsen bewerkstelligt.

## Revendications

1. Procédé de production d'une poudre pharmaceutique pour inhalation, comprenant des particules cristallines d'un composé d'inhalation, comprenant la dissolution d'un composé d'inhalation dans un solvant ; et l'introduction de la solution contenant le composé d'inhalation sous forme de gouttelettes ou sous forme d'un jet, dans un anti-solvant qui est miscible avec le solvant et qui est sous agitation, dans des conditions non super critiques.

2. Procédé selon la revendication 1, dans lequel les particules du composé d'inhalation présentent un diamètre moyen en masse de 10 µm ou moins.

3. Procédé selon la revendication 1, dans lequel les particules du composé d'inhalation présentent un diamètre moyen en masse de 7 µm ou moins.

4. Procédé selon la revendication 1, dans lequel les particules du composé d'inhalation présentent un diamètre moyen en masse d'au moins 0,1 µm.

5. Procédé selon la revendication 1, dans lequel les particules du composé d'inhalation présentent un diamètre moyen en masse d'au moins 1 µm.

6. Procédé selon la revendication 1, dans lequel les particules du composé d'inhalation présentent un diamètre moyen en masse de 1 à 6 µm.

7. Procédé selon la revendication 1, dans lequel au moins 75% de la poudre sont constitués de particules présentant un diamètre de 10 µm ou moins.

8. Procédé selon la revendication 1, dans lequel au moins 90% de la poudre sont constitués de particules présentant un diamètre de 10 µm ou moins.

9. Procédé selon l'une quelconque des revendications précédentes, dans lequel la solution contenant le composé d'inhalation est introduite dans l'anti-solvant à travers un filtre poreux.

10. Procédé selon l'une quelconque des revendications précédentes, dans lequel la solution contenant le composé d'inhalation est introduite dans l'anti-solvant par l'intermédiaire d'une ou de plusieurs buses.

11. Procédé selon l'une quelconque des revendications précédentes, dans lequel la solution contenant le composé d'inhalation est essentiellement saturée ou super saturée.

12. Procédé selon l'une quelconque des revendications précédentes, dans lequel le composé d'inhalation est choisi parmi des agonistes d'adrénorécepteur β2, des bronchodilatateurs anti-cholinergiques, des glucocorticostéroides, des immunomodulateurs, des médicaments anti-allergiques, des expectorants, des mucolytiques, des antihistaminiques, des inhibiteurs de cyclo-oxygénase, des inhibiteurs de la synthèse de leucotriène, des antagonistes de leucotriène, des inhibiteurs de la PLA2, des inhibiteurs de la PKC, des antagonistes du PAF et des agents prophylactiques de l'asthme, et des esters et sels et/ou hydrates pharmacologiquement acceptables de ceux-ci ; et des additifs pharmaceutiquement acceptables.

13. Procédé selon la revendication 12, dans lequel le composé d'inhalation est choisi parmi des agonistes d'adrénorécepteur β2 et des glucocorticostéroïdes.

14. Procédé selon la revendication 13, dans lequel le composé d'inhalation est choisi parmi le salbutamol, le salmétérol, le formotérol, le budésonide, la terbutaline, la fluticasone, le rofléponide, et des esters et sels et/ou solvates pharmaceutiquement acceptables de ceux-ci.

15. Procédé selon la revendication 14, dans lequel le composé d'inhalation est le sulfate de salbutamol.

16. Procédé selon la revendication 14, dans lequel le composé d'inhalation est le dihydrate de fumarate de formotérol.

17. Procédé selon la revendication 14, dans lequel le composé d'inhalation est le budésonide.

18. Procédé selon la revendication 14, dans lequel le composé d'inhalation est le sulfate de terbutaline.

19. Procédé selon la revendication 14, dans lequel le composé d'inhalation est le palmitate de rofléponide.

20. Procédé selon la revendication 14, dans lequel le composé d'inhalation est le xinafoate de salmétérol.

21. Procédé selon l'une quelconque des revendications 1 à 11, dans lequel le composé d'inhalation est un additif pharmaceutiquement acceptable.

22. Procédé selon l'une quelconque des revendications 1 à 11, dans lequel le composé d'inhalation est hydrosoluble, le solvant est l'eau ou un autre solvant polaire ou un mélange de solvants polaires, et l'anti-solvant est un solvant moins polaire.

23. Procédé selon la revendication 22, dans lequel l'anti-solvant est choisi parmi l'acétate d'éthyle, l'acétone, la méthyléthylcétone, l'isopropanol ou des mélanges de 10 à 20% de méthanol, d'isopropanol ou d'éthanol avec 80 à 90% de méthyléthylcétone ou d'isopropanol.

24. Procédé selon l'une quelconque des revendications 1 à 11, dans lequel le composé d'inhalation est essentiellement hydrosoluble, le solvant est moins polaire que l'anti-solvant, et l'anti-solvant est l'eau ou un autre solvant polaire ou un mélange de solvants polaires.

25. Procédé selon la revendication 24, dans lequel le solvant est choisi parmi le méthanol, l'isopropanol, le diméthylsulfoxyde, le diméthylformamide, le N,N'-diméthylacétamide et le phénol.

26. Procédé selon l'une quelconque des revendications précédentes, dans lequel la solution contenant le composé d'inhalation est ajoutée à l'anti-solvant à une température inférieure à 25°C.

27. Procédé selon la revendication 26, dans lequel la solution contenant le composé d'inhalation est ajoutée à l'anti-solvant à une température de 0° à 5°C.

28. Procédé selon l'une quelconque des revendications précédentes, dans lequel l'agitation de l'anti-solvant est réalisée au moyen d'une agitation mécanique, d'hélices, des ailettes de turbines, des roues d'ancrage d'équipement Ystral, ou en employant des ondes ultrasoniques sur ou à côté du filtre ou des buses.
